(19) Europäisches Patentamt
European Patent Office
Office européen des brevets

(11) EP 2 351 849 A1

(12) **EUROPEAN PATENT APPLICATION**
published in accordance with Art. 153(4) EPC

(43) Date of publication:
**03.08.2011 Bulletin 2011/31**

(21) Application number: **09820543.8**

(22) Date of filing: **07.10.2009**

(51) Int Cl.:
*C12Q 1/42* (2006.01) *C12Q 1/26* (2006.01)
*G01N 21/78* (2006.01)

(86) International application number:
**PCT/JP2009/067502**

(87) International publication number:
**WO 2010/044365 (22.04.2010 Gazette 2010/16)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **17.10.2008 JP 2008268871**

(71) Applicant: **Tokyo University of Marine Science and Technology**
**Minato-ku, Tokyo 108-8477 (JP)**

(72) Inventors:
- **SUZUKI, Toru**
  **Tokyo 108-8477 (JP)**
- **HAMADA, Naoko**
  **Tokyo 108-8477 (JP)**
- **SRIRANGSAN, Paveena**
  **Tokyo 108-8477 (JP)**
- **KAWAI, Kiyoshi**
  **Higashihiroshima-shi**
  **Hiroshima 739-0041 (JP)**

(74) Representative: **Fiener, Josef**
**Patentanw. J. Fiener et col.**
**P.O. Box 12 49**
**87712 Mindelheim (DE)**

# (54) REAGENT KIT FOR MEASURING FRESHNESS

(57) A reagent kit for measuring freshness with high storage stability at a relatively high temperature is obtained by lyophilizing reagent solutions each containing a plurality of enzymes and an enzyme protective agent. The reagent kit contains a first reagent and a second reagent. The first reagent is obtained by drying a frozen matter of a first reagent solution containing XOD, NP, an enzyme protective agent, and a color-forming agent under reduced pressure at a temperature not higher than the glass transition point temperature (Tg). The second reagent is obtained by drying a frozen matter of a second reagent solution containing XOD, NP, AP, an enzyme protective agent, and a color-forming agent under reduced pressure at a temperature not higher than the glass transition point temperature (Tg). The enzyme protective agent contains sucrose and/or gelatin. The color-forming agent contains a substance which forms color by being coupled to degradation of Hx into xanthine and uric acid by XOD.

FIG. 5

A
B

EP 2 351 849 A1

## Description

Technical Field

The present invention relates to a reagent kit for measuring freshness. The kit can easily measure freshness of, for example, fish meat in a short time and has high storage stability.

Background Art

Recently, as an index of freshness of, for example, meat, fish meat, or chicken, K value is used. The K value is a ratio (%) of the content of inosine (HxR) and hypoxanthine (Hx) to the total content of adenosine triphosphate (ATP) and ATP decomposition products (ADP, AMP, IMP, HxR, and Hx) in the meat, that is, a numerical value (%) represented by (HxR+Hx)$\times$100/(ATP+ADP+AMP+IMP+HxR+Hx). Herein, ADP denotes adenosine diphosphate, AMP denotes adenosine monophosphate, IMP denotes inosinic acid, HxR denotes inosine, and Hx denotes hypoxanthine.

In order to determine the K value, it is necessary to measure the contents of ATP, ADP, AMP, IMP, HxR, and Hx in meat, fish meat, or chicken. However, the measurement of the contents of these ATP and ATP decomposition products is very troublesome, and in the case of fish meat, ATP, ADP, and AMP are mostly decomposed into IMP (inosinic acid) with time after death of fish, and thereby the amounts of ATP, ADP, and AMP become very low. Accordingly, a numerical value Ki, which is represented by (HxR+Hx)$\times$100/(IMP+HxR+Hx), is assumed as to be Ki$\approx$K and is conveniently used instead of the K value as an index of freshness.

It is known a method for measuring the contents of HxR and Hx as described in, for example, JP-B-62-50120, by mixing a composition solution containing nucleoside phosphorylase (NP), xanthine oxidase (XOD), and a color former with meat juice to decompose HxR in the meat juice to Hx by nucleoside phosphorylase (NP), to decompose Hx to xanthine and uric acid by xanthine oxidase (XOD), and to develop a color by conjugation of the color former with decomposition of Hx, and determining the intensity of the color development.

It is known a method for measuring the contents of IMP, HxR, and Hx as described in, for example, JP-A-Hei-9-262098, by applying a sample solution containing meat juice to a reactor to which alkaline phosphatase (AP), nucleoside phosphorylase (NP), and xanthine oxidase (XOD) are fixed to decompose inosinic acid (IMP) to inosine (HxR) by alkaline phosphatase (AP), to decompose inosine (HxR) to hypoxanthine (Hx) by nucleoside phosphorylase (NP), and to decompose hypoxanthine (Hx) to xanthine and uric acid by xanthine oxidase (XOD), in the sample solution, and measuring the concentration of hypoxanthine (Hx) using a luminous reagent.

However, enzymes (AP, NP, and XOD) used in the above-described methods are very unstable, and their activities easily decrease with time. In addition, their activities further decrease under high temperature. Therefore, it is very difficult to commercialize compositions containing these enzymes.

Against unstableness of these enzymes, for example, JP-A-Hei-8-131196, JP-T-2000-51394, WO 2002/004633, and JP-A-2008-206491 propose some technologies for stabilizing an enzyme by adding sugar (e.g., trehalose or sucrose) or serum albumin (BSA) to an aqueous solution of the enzyme and lyophilizing the solution.

However, there is compatibility between the type of sugar and the type of enzyme for stabilizing the enzyme, and the degree of stabilization of enzyme differs depending on how to combine the enzyme with the sugar. Therefore, the type and concentration of sugar for stabilizing a specific enzyme are not easily determined. In a case of using a mixture of a plurality of enzymes, determination of the type and concentration of sugar for stabilizing these enzymes are further difficult.

When an enzyme is stabilized by freezing an aqueous solution containing the enzyme and then drying it, the conditions, in particular, the conditions in the process for drying the frozen product by increasing the temperature after the freezing also highly affect the stability of the enzyme, but optimum conditions are not known. In a case using a mixture of a plurality of types of enzymes, it is further difficult to determine the optimum conditions.

Citation List

Patent Literature

PTL 1: JP-A-62-50120
PTL 2: JP-A-Hei-9-262098
PTL 3: JP-A-Hei-8-131196
PTL 4: JP-T-2000-513940
PTL 6: WO 2002/004633

PTL 7: JP-A-2008-206491

Summary of Invention

Technical Problem

The problem to be solved is that there is not a reagent kit for measuring freshness, as means for measuring freshness of fish meat, etc., by providing an enzyme activity necessary for appropriately measuring freshness of fish meat, etc. with high storage stability in which enzyme activity is hardly decreased even after storage at high temperature for a long time.

Solution to Problem

The main characteristics of the reagent kit for measuring freshness according to the present invention are that, in order to enhance the storage stability of enzymes contained in the reagent kit for measuring freshness, an enzyme aqueous solution containing an enzyme protecting agent (sugar and/or gelatin) is quickly frozen, and the resulting frozen product is dried at a temperature not higher than the glass transition temperature (Tg) of the frozen product under reduced pressure.

That is, the reagent kit for measuring freshness according to the present invention is composed of a first reagent and a second reagent. The first reagent is a lyophilizate of a first reagent solution containing xanthine oxidase (XOD), nucleoside phosphorylase (NP), an enzyme protecting agent, and a color former, and the second reagent is a lyophilizate of a second reagent solution containing xanthine oxidase (XOD), nucleoside phosphorylase (NP), alkaline phosphatase (AP), an enzyme protecting agent, and a color former.

Herein, the first reagent solution and the second reagent solution are rapidly frozen with, for example, liquid nitrogen and are then dried under reduced pressure. In this case, since the drying time can be shortened by performing the drying while increasing sample temperature (lyophilizer shelf temperature) with elapse of time, the temperature of the solution is preferably increased with elapse of time during the drying of the solution. However, since the enzyme activity decreases if the solution temperature is increased to a temperature not lower than the glass transition temperature (Tg) of the product obtained by the drying, the sample temperature should be increased in the range not higher than the glass transition temperature (Tg) of the product obtained by the drying.

As the enzyme protecting agent, sucrose and/or gelatin can be used. The concentration of sucrose is preferably in the range of 50 to 400 mM. A sucrose concentration in this range can provide a reagent having high storage stability. The concentration of gelatin is preferably in the range of 0.1 to 2.0 (grams of gelatin/100 mL of reagent solution). A gelatin concentration in this range can provide a reagent having high storage stability.

As the color former, one that develops a color by conjugation with a reaction of decomposing hypoxanthine (Hx) into xanthine and uric acid by xanthine oxidase (XOD) can be used, and examples thereof include known formazan reagents such as tetrazolium blue (TB), nitrotetrazolium blue (nitro-TB), tetrazolium violet (TV), nitroblue tetrazolium (NBT), 3-(4,5-dimethyl-2-thiazolyl)-2,5-diphenyl-2H-tetrazolium (MTT), 2-(4-iodophenyl)-3-(4-nitrophenyl)-5-(2,4-disulphenyl)-2H-t etrazolium salt (WST-1), 2-(4-iodophenyl)-3-(2,4-dinitrophenyl)-5-(2,4-disulphenyl)-2H-tetrazolium salt (WST-3), 2-(2-methoxy-4-nitrophenyl)-3-(4-nitrophenyl)-5-(2,4-disulp henyl)-2H-tetrazolium salt (WST-8), 2,3,5-triphenyltetrazolium, and 3-(4,5-dimethylthiazol-2-phenyl)-5-(3-carboxymethoxyphenyl) -2-(4-sulfophenyl)-2H-tetrazolium salt (MTS).

The concentration of the color former is preferably in the range of 0.1 to 0.6 mM when the color former is WST-8. A color former concentration in this range can give stable color development. In a color former concentration higher than 0.6 mM, the intensity of color development reaches the peak level, which is economically wasteful. Accordingly, the concentration of 0. 6 mM has been determined to be the upper limit, but the concentration may be higher than 0.6 mM.

In each of the first reagent solution and the second reagent solution, the concentration of the xanthine oxidase (XOD) is preferably in the range of 0.1 to 10 U/mL, and the concentration of the nucleoside phosphorylase (NP) is preferably in the range of 0.05 to 20 U/mL. In the second reagent solution, the concentration of the alkaline phosphatase (AP) is preferably in the range of 5 to 200 U/mL.

The lower limit concentrations of the xanthine oxidase (XOD), the nucleoside phosphorylase (NP), and the alkaline phosphatase (AP) have been determined to be 0.1 U/mL, 0.05 U/mL, and 5 U/ml, respectively, because the stabilities of these enzymes are decreased at concentrations lower than these levels. The upper limit concentrations of the xanthine oxidase (XOD), the nucleoside phosphorylase (NP), and the alkaline phosphatase (AP) have been determined to be 10 U/mL, 20 U/mL, and 200 U/mL, respectively, because the storage stabilities of the reagents are decreased at concentrations higher than these levels.

The first reagent solution and the second reagent solution may or may not contain serum albumin (BSA).

The first reagent solution and the second reagent solution preferably each have a pH of 6.8 to 8.5, in which the above-mentioned enzymes can function. The pH levels of the first reagent solution and the second reagent solution are preferably adjusted in the range of 6.8 to 8.5 with a buffer such as potassium phosphate.

The first reagent solution and the second reagent solution may be each impregnated in paper and then lyophilized to give two types of test papers that can be used for measuring freshness of fish meat, etc.

Advantageous Effects of Invention

In the present invention, each reagent is prepared by freezing a reagent solution dissolving the above-described two or three types of enzymes and an enzyme protecting agent, and drying the resulting frozen product under reduced pressure in a temperature range of not higher than the glass transition temperature (Tg). This provides advantages that the storage stability (remaining activity) of each reagent has been increased and that each reagent can be stored at relatively high temperature for a long time.

In addition, since each reagent can be stored at relatively high temperature (for example, 55°C) over a long period of time, the kit can be used, for example, for raw material quality inspection in seafood processing factories and food factories using seafood products. This provides an advantage that freshness of individual fish can be objectively determined on site.

Furthermore, each reagent can be stored at relatively high temperature (for example, 55°C) over a long period of time. This provides an advantage that the kit can be simply used for determining freshness of fish in areas not having refrigeration facilities, such as developing countries.

Brief Description of Embodiments

[Fig. 1] Fig. 1 is a graph showing a relationship between [HxR+Hx] concentration (μM) and absorbance (454 nm).

[Fig. 2] Fig. 2 is a graph showing a relationship between [IMP+HxR+Hx] concentration (μM) and absorbance (454 nm).

[Fig. 3] Fig. 3 is a graph showing a correlation between Ki value determined by a reagent kit of the present invention and Ki value determined by HPLC.

[Fig. 4] Fig. 4 is a graph showing a relationship between storage period of a reagent kit of the present invention and Ki value.

[Fig. 5] Fig. 5 is an explanatory diagram showing a method image of a reagent kit of the present invention.

[Fig. 6] Fig. 6 is a graph showing a relationship between storage period (day) and remaining activity of the first reagent stored at 25°C.

[Fig. 7] Fig. 7 is a graph showing a relationship between storage period (day) and remaining activity of the second reagent stored at 25°C.

[Fig. 8] Fig. 8 is a graph showing a relationship between storage period (day) and remaining activity of the first reagent stored at 40°C.

[Fig. 9] Fig. 9 is a graph showing a relationship between storage period (day) and remaining activity of the second reagent stored at 40°C.

[Fig. 10] Fig. 10 is a graph showing a relationship between storage period (day) and remaining activity of the second reagent stored at 55°C.

[Fig. 11] Fig. 11 is a graph showing a relationship between storage period (day) and remaining activity of the second reagent stored at 55°C.

[Fig. 12] Fig. 12 is a graph showing a relationship between storage period (day) and remaining activity of the first reagent at each humidity.

[Fig. 13] Fig. 13 is a graph showing a relationship between storage period (day) and remaining activity of the second reagent at each humidity.

[Fig. 14] Fig. 14 is a graph showing a relationship between storage period (day) of an enzyme composite (reagent kit) not containing an enzyme protecting agent and Ki value of fish meat measured by the reagent kit using the same fish meat on each day of the storage period.

[Fig. 15] Fig. 15 is a graph showing a relationship between storage period (day) of an enzyme composite (reagent kit) containing sucrose and Ki value of fish meat measured by the reagent kit using the same fish meat on each day of the storage period.

[Fig. 16] Fig. 16 is a graph showing a relationship between storage period (day) of an enzyme composite (reagent kit) containing sucrose and gelatin and Ki value of fish meat measured by the reagent kit using the same fish meat on each day of the storage period.

Description of Embodiments

The object of providing a reagent kit for measuring freshness, the storage stability of the kit is high, has been realized by a simple method without reducing basic activities of the enzymes.

Example 1

Nucleoside phosphorylase (NP) was dissolved in 20 mM potassium phosphate buffer (pH = 7.8) to prepare a NP solution. Xanthine oxidase (XOD) was dissolved in 20 mM potassium phosphate buffer (pH = 7.8) to prepare a XOD solution. Then, stabilizers contained in the NP solution and the XOD solution were removed by dialysis. Sucrose was dissolved in 20 mM potassium phosphate buffer (pH = 7.8) to prepare a sucrose solution. WST-8 (color former) was dissolved in 20 mM potassium phosphate buffer (pH = 7.8) to prepare a WST-8 (color former) solution.

Then, all of these NP solution, XOD solution, sucrose solution, and WST-8 (color former) solution were mixed to prepare a first reagent solution. This first reagent solution was put in a 2-mL polypropylene tube and immersed in liquid nitrogen for one minute to rapidly freeze the solution. Then, this polypropylene tube was put in a lyophilizer, in a state that the upper lid was opened, for drying. The drying was performed under a reduced pressure of $3.0 \times 10^{-2}$ Torr. On this occasion, the temperature was increased from -40°C to 5°C by 5°C for each 3 hours, subsequently, from 5°C to 25°C by 10°C for each 3 hours. Then, the dried product was taken out from the lyophilizer and was transferred to a desiccator containing $P_2O_5$ and stored therein for 7 days for complete dehydration to obtain a first reagent.

Nucleoside phosphorylase (NP) was dissolved in 20 mM potassium phosphate buffer (pH = 7.8) to prepare a NP solution. Xanthine oxidase (XOD) was dissolved in 20 mM potassium phosphate buffer (pH = 7.8) to prepare a XOD solution. An alkaline phosphatase (AP) solution was prepared. Then, stabilizers contained in the NP solution, the XOD solution, and the AP solution were removed by dialysis. Sucrose was dissolved in 20 mM potassium phosphate buffer (pH = 7.8) to prepare a sucrose solution. WST-8 (color former) was dissolved in 20 mM potassium phosphate buffer (pH = 7.8) to prepare a WST-8 (color former) solution.

Then, all of these NP solution, XOD solution, AP solution, sucrose solution, and WST-8 (color former) solution were mixed to prepare a second reagent solution. This second reagent solution was put in a 2-mL polypropylene tube and immersed in liquid nitrogen for one minute to rapidly freeze the solution. Then, this polypropylene tube was put in a lyophilizer, in a state that the upper lid was opened, for drying. The drying was performed under a reduced pressure of $3.0 \times 10^{-2}$ Torr. On this occasion, the temperature was increased from -40°C to 5°C by 5°C for each 3 hours, subsequently, from 5°C to 25°C by 10°C for each 3 hours. Then, the dried product was taken out from the lyophilizer and was transferred to a desiccator containing $P_2O_5$ and stored therein for 7 days for complete dehydration to obtain a second reagent.

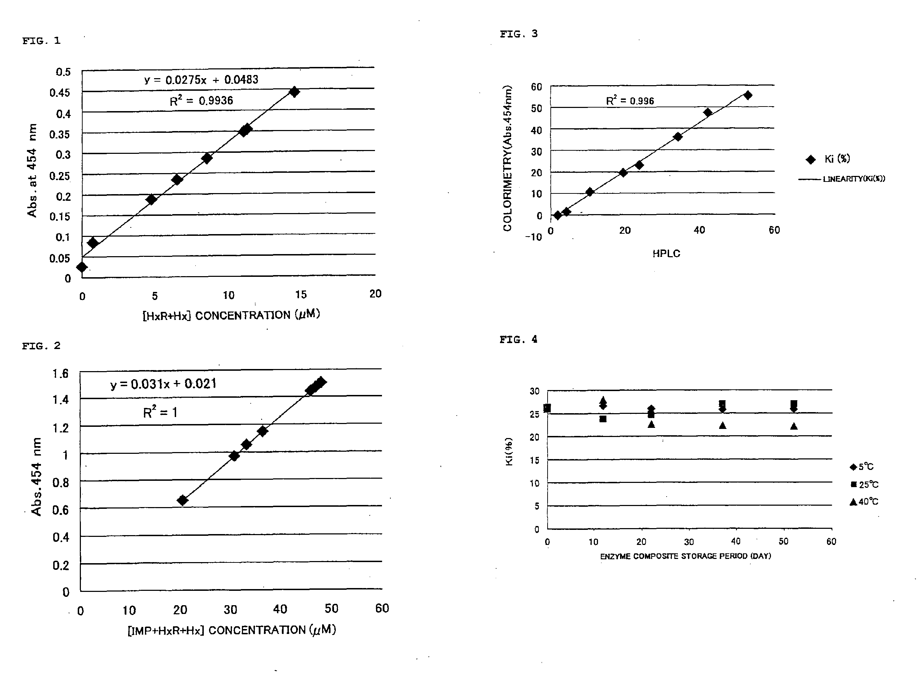

Then, 60 mg of the first reagent was dissolved in 1 mL of distilled water, and a fraction of 150 μL of the resulting solution was mixed with 150 μL of squeezed juice of fish meat. The absorbance A of the solution mixture was measured at 454 nm with a spectrophotometer. Then, as shown in Fig. 1, the [HxR+Hx] concentration A (μM) in the squeezed juice of fish meat was determined from the relationship between absorbance (Abs. at 454 nm) and [HxR+Hx] concentration (μM) prepared in advance.

Separately, 60 mg of the second reagent was dissolved in 1 mL of distilled water, and a fraction of 150 μL of the resulting solution was mixed with 150 μL of squeezed juice of fish meat. The absorbance A of the solution mixture was measured at 454 nm with a spectrophotometer. Then, as shown in Fig. 2, the [IMP+HxR+Hx] concentration B (μM) in the squeezed juice of fish meat was determined from the relationship between absorbance (Abs. at 454 nm) and [IMP+HxR+Hx] concentration (μM) prepared in advance.

Then, Ki value (= A/B) was determined based on $(HxR+Hx)\times 100/(IMP+HxR+Hx) = Ki(\%)$, wherein (HxR+Hx) is the concentration A, and (IMP+HxR+Hx) is the concentration B.

Separately, the Ki value of the same fish meat was determined by high-performance liquid chromatography (HPLC). A correlation between the Ki value determined by the reagent kit of the present invention and the Ki value determined by HPLC was obtained. The results were as shown by the graph in Fig. 3. It can be confirmed from the results shown by the graph in Fig. 3 that there is a high correlation, R2 = 0.996, between the Ki value determined by the measuring method of the present invention and the Ki value determined by HPLC.

Example 2

The reagent kits of the present invention were stored at 5°C, 25°C, or 40°C for 50 days, and the Ki value of fish meat (Japanese horse mackerel) was measured by each of the kits. The results are shown in Fig. 4. It can be confirmed from the results that the reagent kit of the present invention can be stored at 40°C for 50 days in a state maintaining the enzyme activities considerably high.

Example 3

In an experiment as in Example 1, when the concentration of alkaline phosphatase (AP) was less than 5 U/mL, the resulting Ki values were unstable, which may be caused by that decomposition of IMP by alkaline phosphatase (AP) was incomplete, and when the concentration of alkaline phosphatase (AP) was higher than 200 U/mL, the storage stability of the reagent was decreased. However, when the concentration of alkaline phosphatase (AP) was from 5 to 200 U/mL, such disadvantages did not occur to give stable Ki values. Accordingly, the optimum concentration range of alkaline phosphatase (AP) is judged to be from 5 to 200 U/mL.

Example 4

In an experiment as in Example 1, when the concentration of nucleoside phosphorylase (NP) was less than 0.05 U/mL, the resulting Ki values were unstable, which may be caused by that decomposition of HxR by nucleoside phosphorylase (NP) was incomplete, and when the concentration of nucleoside phosphorylase (NP) was higher than 20 U/mL, the storage stability of the reagent was decreased. However, when the concentration of nucleoside phosphorylase (NP) was from 0.05 to 20 U/mL, such disadvantages did not occur to give stable Ki values. Accordingly, the optimum concentration range of nucleoside phosphorylase (NP) is judged to be from 0.05 to 20 U/mL.

Example 5

In an experiment as in Example 1, when the concentration of xanthine oxidase (XOD) was less than 0.1 U/mL, the resulting Ki values were unstable, which may be caused by that decomposition of Hx by xanthine oxidase (XOD) was incomplete, and when the concentration of xanthine oxidase (XOD) was higher than 10 U/mL, the storage stability of the reagent was decreased. However, when the concentration of xanthine oxidase (XOD) was from 0.1 to 10 U/mL, such disadvantages did not occur to give stable Ki values. Accordingly, the optimum concentration range of xanthine oxidase (XOD) is judged to be from 0.1 to 10 U/mL.

Example 6

In an experiment as in Example 1, when the concentration of sucrose was less than 50 mM, the resulting Ki values were unstable, and when the concentration of sucrose was higher than 400 mM, the enzyme solution was crystallized. However, when the concentration of sucrose was from 50 to 400 mM, such disadvantages did not occur to give stable Ki values. Accordingly, the optimum concentration range of sucrose is judged to be from 50 to 400 mM.

Example 7

In an experiment as in Example 1, when the concentration of WST-8 (color former) was less than 0.1 mM, the color development was unstable, and when the concentration of WST-8 (color former) was higher than 0.6 mM, the degree of color development reached the peak level regardless of an increase in concentration. However, when the concentration of WST-8 (color former) was from 0.1 to 0.6 mM, such disadvantages did not occur to give stable Ki values. Accordingly, the optimum concentration range of WST-8 (color former) is judged to be from 0.1 to 0.6 mM.

Example 8

In an experiment as in Example 1, when the pH was lower than 6.8 or higher than 8.5, the enzymes did not function, but when the pH was from 6.8 to 8.5, such disadvantage did not occur to give stable Ki values. Accordingly, the optimum pH range is judged to be from 6.8 to 8.5.

Example 9

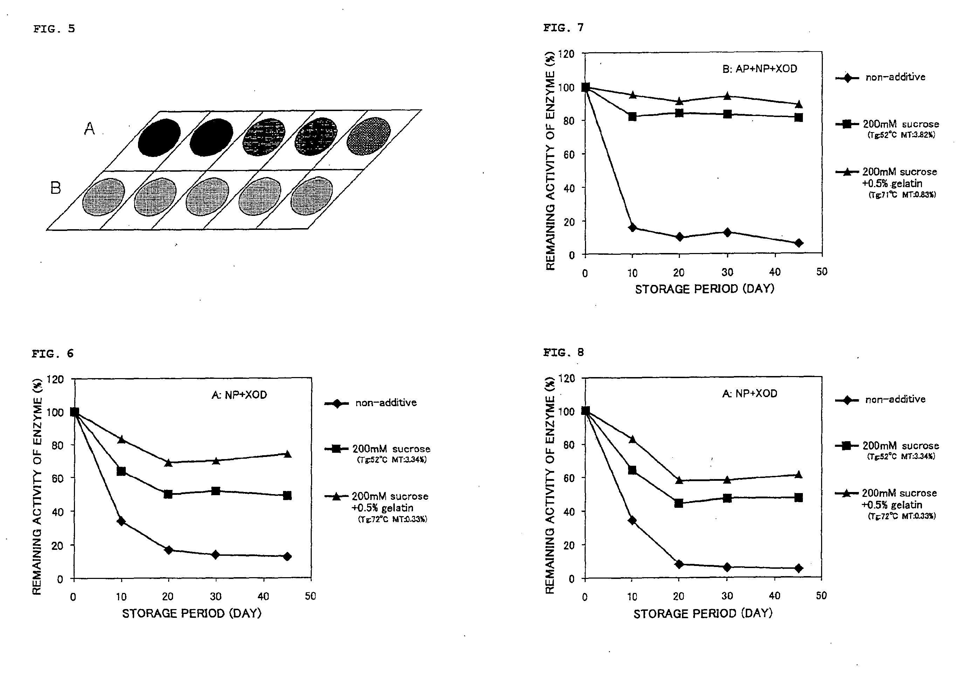

In the above-described examples, the color intensity of a reagent was determined using an absorptiometer, but, as shown in Fig. 5, the [IMP+HxR+Hx] concentration or the [HxR+Hx] concentration may be determined by lyophilizing each reagent, dispensing the resulting powder to each well of a plate reader, adding an appropriate amount of fish meat extraction to the wells to develop a color, and comparing the color intensity with color samples. Alternatively, an enzyme solution lyophilized in a state being dispensed to each well of a plate reader may be used as a reagent kit.

Example 10

A first reagent solution containing 0.3 U/mL of nucleoside phosphorylase (NP) and 0.6 U/mL of xanthine oxidase (XOD) was prepared by dissolving 0.02 mg of NP and 2 mg of XOD in 1 mL of a solution containing 200 mM sucrose or in 1 mL of a solution containing 200 mM sucrose and 0.5% gelatin.

A second reagent solution containing 45 U/mL of alkaline phosphatase (AP), 0.3 U/mL of nucleoside phosphorylase (NP), and 0.6 U/mL of xanthine oxidase (XOD) was prepared by dissolving 1.5 μL of AP, 0.02 mg of NP, and 2 mg of XOD in 1 mL of a solution containing 200 mM sucrose or in 1 mL of a solution containing 200 mM sucrose and 0.5% gelatin.

One milliliter of the first reagent solution was put in a 2-mL polypropylene tube, and 1 mL of the second reagent solution was put in another 2-mL polypropylene tube. These polypropylene tubes were immersed in liquid nitrogen for at least one minute to rapidly freeze the solutions. The resulting frozen products were stored at -90°C.

Then, each frozen product was transferred to a lyophilizer cooled in advance to -40°C, and the temperature was increased from -40°C to 5°C by 5°C for each step, subsequently, from 5°C to 25°C by 10 °C for each step, while maintaining the temperature in each step for 3 hours, to dry the frozen product. In this procedure, the pressure in the lyophilizer was maintained at $3.0 \times 10^{-2}$ Torr over the drying process. The frozen product lost its water by the lyophilization to form a dried solid.

The remaining water in the dried solid was further removed in a vacuum desiccator over $P_2O_5$ at room temperature for 7 days. The dried solid obtained from the frozen product of the first reagent solution and the dried solid obtained from the frozen product of the second reagent solution were placed in a glove box replaced with dried nitrogen as a first reagent and a second reagent, respectively, and were stored at 25°C, 40°C, or 55°C for 45 days.

Then, 60 mg of the first reagent was dissolved in 1 mL of distilled water. To a fraction of 75 μL of the resulting solution, 75 μL of 4 mM HxR and then 150 μL of 20 mM potassium phosphate buffer were added to prepare a first reagent solution. The absorbance at 292 nm of the first reagent solution was measured at 20°C using a UV-VIS spectrophotometer. The enzyme activity was evaluated by comparing it with the initial activity.

Similarly, 60 mg of the second reagent was dissolved in 1 mL of distilled water. To a fraction of 75 μL of the resulting solution, 75 μL of 4 mM HxR and 1 mM $MgCl_2$ and then 150 μL of 20 mM potassium phosphate buffer were added to prepare a second reagent solution. The absorbance at 292 nm of the second reagent solution was measured at 20°C using a UV-VIS spectrophotometer. The enzyme activity was evaluated by comparing it with the initial activity. The remaining activity was expressed by percentage with respect to the activity before the lyophilization.

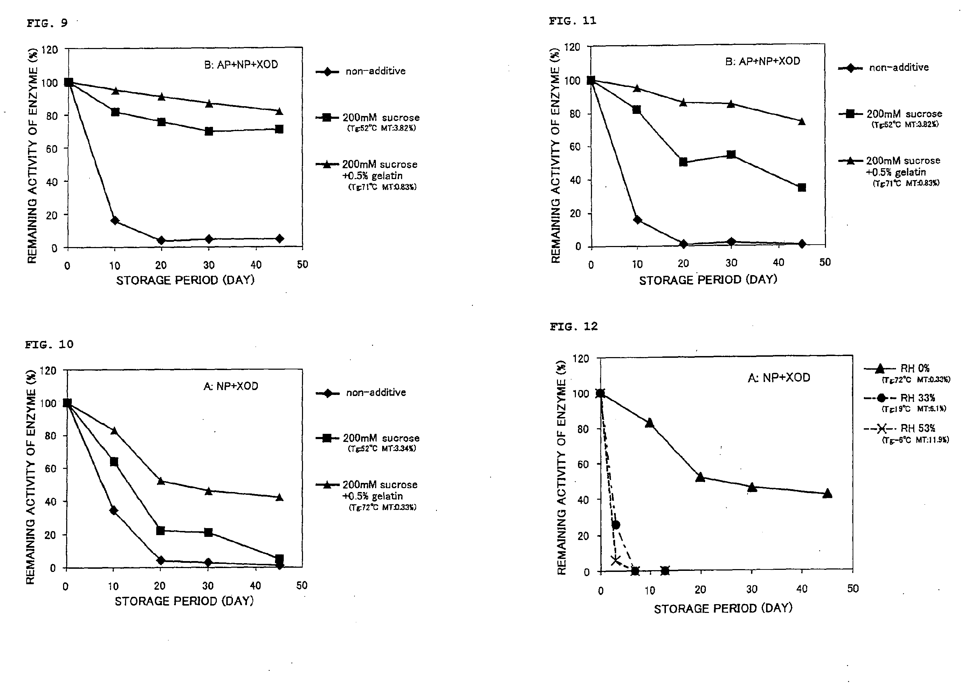

The remaining activities of the enzymes in the first reagent stored at a storage temperature of 25°C for from 0 to 45 days were as shown in Fig. 6; the remaining activities of the enzymes in the second reagent stored at a storage temperature of 25°C for from 0 to 45 days were as shown in Fig. 7; the remaining activities of the enzymes in the first reagent stored at a storage temperature of 40°C for from 0 to 45 days were as shown in Fig. 8; the remaining activities of the enzymes in the second reagent stored at a storage temperature of 40°C for from 0 to 45 days were as shown in Fig. 9; the remaining activities of the enzymes in the first reagent stored at a storage temperature of 55°C for from 0 to 45 days were as shown in Fig. 10; and the remaining activities of the enzymes in the second reagent stored at a storage temperature of 55°C for from 0 to 45 days were as shown in Fig. 11.

It can be confirmed from the results shown in Figs. 6 to 11 that the remaining activities of the first reagent and the second reagent containing sucrose were increased in every cases compared with those not containing sucrose and that the remaining activities of the reagents containing both sucrose and gelatin were further increased in every cases compared with those containing only sucrose. Note that the water contents of the first reagent and the second reagent were measured with a Karl Fischer moisture titrator (737 KF, Herisau, Switzerland) and that the heat characteristics of the first reagent and the second reagent were investigated with a differential scanning calorimeter (DSC-50, Shimadzu, Japan).

Example 11

A first reagent containing sucrose and gelatin and a second regent containing sucrose and gelatin were prepared and were stored at 55°C for from 0 to 45 days in a relative humidity environment of 0%, 33%, or 53%. The remaining activity of each reagent in each humidity environment was investigated on each day of the storage period. Herein, each reagent was produced by the same method as in Example 10, and the remaining activity of each reagent was measured by the same method as in Example 10.

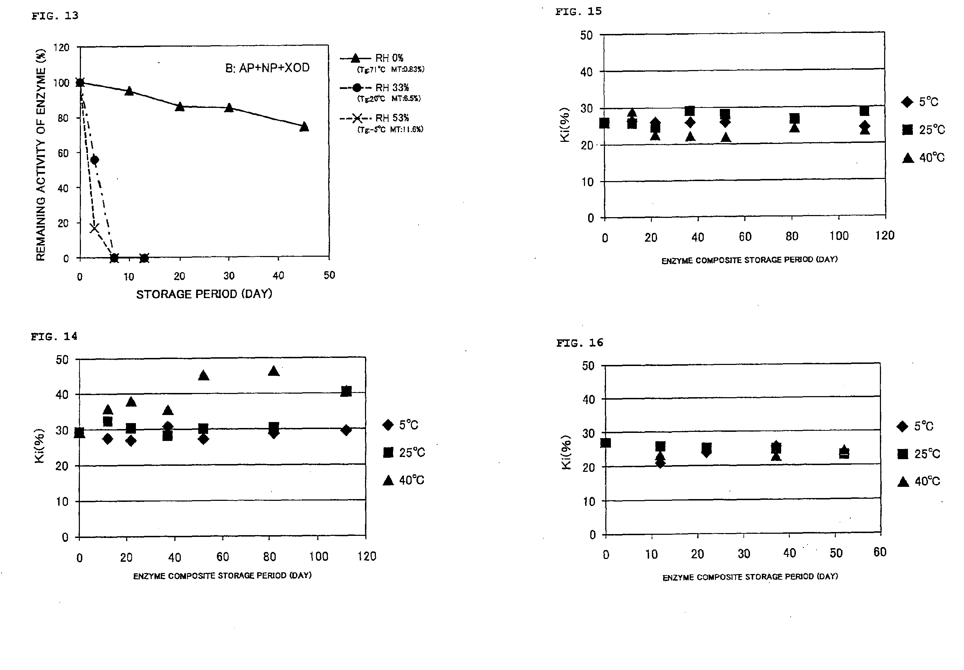

The remaining activity of the first reagent in each humidity environment was as shown in Fig. 12, and the remaining activity of the second reagent in each humidity environment was as shown in Fig. 13. It can be confirmed from the results shown in Figs. 12 and 13 that the remaining activity in a relative humidity of 0% is significantly high compared with that in relative humidity of 33% or 53%. It can be confirmed that in order to prevent a decrease in remaining activity of a reagent, it is necessary to store the reagent in an atmosphere in which the relative humidity has been reduced as much as possible so as to be near 0%.

Example 12

A first reagent not containing sucrose and gelatin, a first reagent (Tg: 65°C, MT: 0.81%) containing sucrose but not containing gelatin, and a first reagent (Tg: 77°C, MT: 0.5%) containing both sucrose and gelatin were prepared and stored at 5°C, 25°C, or 40°C for from 0 to 112 days.

A second reagent not containing sucrose and gelatin, a second reagent (Tg: 65°C, MT: 0.78%) containing sucrose but not containing gelatin, and a second reagent (Tg: 76°C, MT: 0.99%) containing both sucrose and gelatin were prepared and stored at 5°C, 25°C, or 40°C for from 0 to 112 days. Herein, the fundamental compositions and conditions for preparing the first reagents and the second reagents were the same as those in Example 10, and a color former, WST-8, was added to each reagent.

Inosine (HxR) standard solutions were prepared by adding 0, 5, 10, 20, 30, or 50 μM HxR to 20 mM potassium phosphate buffer. Inosinic acid (IMP) standard solutions were prepared by adding 0, 5, 10, 20, 30, or 50 μM IMP and 1 mM $MgCl_2$ to 20 mM potassium phosphate buffer. The actual HxR concentrations in the HxR standard solutions and the actual IMP concentrations in the IMP standard solutions were determined by high-performance liquid chromatography (HPLC).

The first reagent solution (150 μL) was added to 150 μL of each of the HxR (0, 5, 10, 20, 30, and 50 μM) standard solutions, and the absorbance at 454 nm of each solution was measured with a UV-VIS spectrophotometer at 25°C to determine a relationship between the HxR concentration and the absorbance, and a standard curve was prepared based on the relationship. The second reagent solution (150 μL) was added to 150 μL of each of the IMP (0, 5, 10, 20, 30, and 50 μM) standard solutions, and the absorbance at 454 nm of each solution was measured with a UV-VIS spectrophotometer at 25°C to determine a relationship between the IMP concentration and the absorbance, and a standard curve was prepared based on the relationship.

Fish meat (1 g) was mixed with 10% perchloric acid (4 mL) while mincing, and 5% perchloric acid (4 mL) was further added thereto and mixed. The resulting mixture was centrifuged at a speed of 2000 rpm at 5 °C for 10 minutes. The supernatant was collected and was adjusted its pH to 6.8 to 7.0 with 8 M or 1 M KOH. The supernatant was centrifuged again at a speed of 2000 rpm at 5°C for 10 minutes. The sample solution was filtered through Watman No. 1 filter paper and diluted with distilled water graded by high-performance liquid chromatography (HPLC) to obtain 20 mL of a fish extract. The diluted sample solution was filtered again through millipore paper with a pore size of 0.65 μm and stored at -90°C until measurement of freshness.

Then, the Ki values of the same fish meat sample on each day of the storage period and at each storage temperature were investigated using the first reagent and the second reagent. Herein, the Ki value of the fish meat sample was determined as follows: First, the first reagent and the second reagent (60 mg each) were each dissolved in 1 mL of distilled water, and to 150 μL of each of the resulting solutions, the fish extract (20 μL) and 20 mM potassium phosphate buffer (130 μL) were added to prepare a first reagent solution and a second reagent solution.

The absorbance at 454 nm of each of the first reagent solution and the second reagent solution was measured with a UV-VIS spectrophotometer at 25°C, and the concentration of HxR+Hx and the concentration of IMP+HxR+Hx were respectively determined using the results obtained by the first reagent solution and the second reagent solution based on the standard curves. Then, the Ki value was determined by fitting the concentration of HxR+Hx and the concentration of IMP+HxR+Hx to the equation shown by the following Expression 1:

[Expression 1]

$$Ki(\%) = (HxR + Hx) \times 100 / (IMP + HxR + Hx)$$

The Ki values determined using the enzyme composite (first reagent and second reagent) not containing sucrose and gelatin were as shown in Fig. 14; the Ki values determined using the enzyme composite (first reagent and second reagent) containing only sucrose were as shown in Fig. 15; and the Ki values determined using the enzyme composite (first reagent and second reagent) containing sucrose and gelatin were as shown in Fig. 16.

It can be confirmed from the results shown in Figs. 14 to 16 that the variation in the Ki values determined using the enzyme composite (first reagent and second reagent) containing sucrose was small compared to that in the Ki values determined using the enzyme composite (first reagent and second reagent) not containing sucrose and gelatin and that the variation in the Ki values determined using the enzyme composite (first reagent and second reagent) containing sucrose and gelatin was further small.

## Claims

1. A reagent kit for measuring freshness, comprising a first reagent and a second reagent, wherein the first reagent comprises a product obtained by drying a frozen product of a first reagent solution containing xanthine oxidase (XOD), nucleoside phosphorylase (NP), an enzyme protecting agent, and a color former under reduced pressure at a temperature not higher than the glass transition temperature (Tg); and the second reagent comprises a product obtained by drying a frozen product of a second reagent solution containing xanthine oxidase (XOD), nucleoside phosphorylase (NP), alkaline phosphatase (AP), an enzyme protecting agent, and a color former under reduced pressure at a temperature not higher than the glass transition temperature (Tg), wherein the enzyme protecting agents are each sucrose and/or gelatin; and the color formers each develop a color by conjugation with a reaction of decomposing hypoxanthine (Hx) into xanthine and uric acid by xanthine oxidase (XOD).

2. The reagent kit for measuring freshness according to Claim 1, wherein the temperature for reduced-pressure drying of the frozen product of the first reagent solution is increased with elapse of time, and the temperature for reduced-pressure drying of the frozen product of the second reagent solution is increased with elapse of time.

3. The reagent kit for measuring freshness according to Claim 1, wherein the first reagent solution includes xanthine oxidase (XOD) in a concentration range of 0.1 to 10 U/mL and nucleoside phosphorylase (NP) in a concentration range of 0.05 to 20 U/mL; and the second reagent solution includes xanthine oxidase (XOD) in a concentration range of 0.1 to 10 U/mL, nucleoside phosphorylase (NP) in a concentration range of 0.05 to 20 U/mL, and alkaline phosphatase (AP) in a concentration range of 5 to 200 U/mL.

4. The reagent kit for measuring freshness according to Claim 3, wherein the first reagent solution and the second reagent solution each contain sucrose in a concentration range of 50 to 400 mM.

5. The reagent kit for measuring freshness according to Claim 3, wherein the first reagent solution and the second reagent solution each contain gelatin in a concentration range of 0.1 to 2.0 (g of gelatin/100 mL of reagent solution).

6. The reagent kit for measuring freshness according to Claim 3, wherein the first reagent solution and the second reagent solution each contain the color former in the concentration range of 0.1 to 0.6 mM.

7. The reagent kit for measuring freshness according to Claim 3, wherein the first reagent solution and the second reagent solution each have a pH of 6.8 to 8.5.

8. The reagent kit for measuring freshness according to Claim 3, wherein the color former is WST-8.

9. The reagent kit for measuring freshness according to Claim 1, wherein the frozen product of the first reagent solution is prepared by rapidly freezing the first reagent solution with liquid nitrogen, and the frozen product of the second reagent solution is prepared by rapidly freezing the second reagent solution with liquid nitrogen.

10. The reagent kit for measuring freshness according to Claim 1, wherein the first reagent solution and the second reagent solution each contain serum albumin (BSA).

FIG. 1

y = 0.0275x + 0.0483
$R^2$ = 0.9936
Abs. at 454 nm
[HxR+Hx] CONCENTRATION (μM)
0.5
0.45
0.4
0.35
0.3
0.25
0.2
0.15
0.1
0.05
0
5
10
15
20

FIG. 2

y = 0.031x + 0.021
$R^2$ = 1
Abs.454 nm
[IMP+HxR+Hx] CONCENTRATION (μM)
1.6
1.4
1.2
1
0.8
0.6
0.4
0.2
0
10
20
30
40
50
60

FIG. 3

$R^2$ = 0.996
COLORIMETRY(Abs.454nm)
HPLC
Ki (%)
LINEARITY(Ki(%))
60
50
40
30
20
10
0
-10
20
40
60

FIG. 4

Ki(%)
ENZYME COMPOSITE STORAGE PERIOD (DAY)
5°C
25°C
40°C
30
25
20
15
10
5
0
10
20
30
40
50
60

FIG. 5

A
B

FIG. 6

A: NP+XOD
REMAINING ACTIVITY OF ENZYME (%)
STORAGE PERIOD (DAY)
0
20
40
60
80
100
120
10
30
50
non-additive
200mM sucrose
(Tg:52°C MT:3.34%)
200mM sucrose
+0.5% gelatin
(Tg:72°C MT:0.33%)

FIG. 7

B: AP+NP+XOD
REMAINING ACTIVITY OF ENZYME (%)
STORAGE PERIOD (DAY)
0
20
40
60
80
100
120
10
30
50
non-additive
200mM sucrose
(Tg:52°C MT:3.82%)
200mM sucrose
+0.5% gelatin
(Tg:71°C MT:0.83%)

FIG. 8

A: NP+XOD
REMAINING ACTIVITY OF ENZYME (%)
STORAGE PERIOD (DAY)
0
20
40
60
80
100
120
10
30
50
non-additive
200mM sucrose
(Tg:52°C MT:3.34%)
200mM sucrose
+0.5% gelatin
(Tg:72°C MT:0.33%)

FIG. 9

B: AP+NP+XOD
non-additive
200mM sucrose
200mM sucrose
+0.5% gelatin
REMAINING ACTIVITY OF ENZYME (%)
STORAGE PERIOD (DAY)

FIG. 10

A: NP+XOD
non-additive
200mM sucrose
200mM sucrose
+0.5% gelatin
REMAINING ACTIVITY OF ENZYME (%)
STORAGE PERIOD (DAY)

FIG. 11

B: AP+NP+XOD
non-additive
200mM sucrose
200mM sucrose
+0.5% gelatin
REMAINING ACTIVITY OF ENZYME (%)
STORAGE PERIOD (DAY)

FIG. 12

A: NP+XOD
RH 0%
RH 33%
RH 53%
REMAINING ACTIVITY OF ENZYME (%)
STORAGE PERIOD (DAY)

FIG. 13

B: AP+NP+XOD
RH 0% (Tg:71°C MT:0.83%)
RH 33% (Tg:20°C MT:6.5%)
RH 53% (Tg:-5°C MT:11.6%)
REMAINING ACTIVITY OF ENZYME (%)
STORAGE PERIOD (DAY)

FIG. 14

5°C
25°C
40°C
Ki(%)
ENZYME COMPOSITE STORAGE PERIOD (DAY)

FIG. 15

5°C
25°C
40°C
Ki(%)
ENZYME COMPOSITE STORAGE PERIOD (DAY)

FIG. 16

5°C
25°C
40°C
Ki(%)
ENZYME COMPOSITE STORAGE PERIOD (DAY)

## INTERNATIONAL SEARCH REPORT

International application No. PCT/JP2009/067502

A. CLASSIFICATION OF SUBJECT MATTER
*C12Q1/42*(2006.01)i, *C12Q1/26*(2006.01)i, *G01N21/78*(2006.01)i

According to International Patent Classification (IPC) or to both national classification and IPC

B. FIELDS SEARCHED

Minimum documentation searched (classification system followed by classification symbols)
C12Q1/42, C12Q1/26, G01N21/78

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
CAplus/BIOSIS/MEDLINE/WPIDS(STN), JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
|---|---|---|
| Y/A | JP 1-289500 A (Daiichi Pure Chemicals Co., Ltd.), 21 November 1989 (21.11.1989), page 4, upper right column, line 19 to lower left column, line 21; page 6, lower left column, line 16 to page 6, lower right column, line 19 & EP 383922 A1 & WO 1989/007655 A1 | 1-2,9-10/3-8 |
| Y/A | JP 2-265984 A (Pafra Ltd.), 30 October 1990 (30.10.1990), page 2, lower left column, line 19 to lower right column, line 8 & US 5098893 A & US 37872 E & US 38385 E & US 39497 E & EP 383569 A2 | 1-2,9-10/3-8 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 29 October, 2009 (29.10.09) | 10 November, 2009 (10.11.09) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.
PCT/JP2009/067502

C (Continuation). DOCUMENTS CONSIDERED TO BE RELEVANT

| Category* | Citation of document, with indication, where appropriate, of the relevant passages | Relevant to claim No. |
|---|---|---|
| Y/A | JP 11-504802 A (Kairon Corp.), 11 May 1999 (11.05.1999), page 87, lines 19 to 27 & US 5789245 A & US 5814482 A & US 5843723 A & US 6015686 A & US 6015694 A & US 6342372 B1 & US 6376236 B1 & US 2003/0232035 A1 & US 2004/0029278 A1 & EP 797679 A2 & WO 1996/017072 A2 | 1-2,9-10/3-8 |
| Y/A | JP 2000-513940 A (Molecular Biology Resources, Inc.), 24 October 2000 (24.10.2000), page 13, line 7 to page 14, line 12 & US 5876992 A & US 6294365 B1 & EP 910630 A1 & WO 1998/000530 A1 | 1-2,9-10/3-8 |
| A | JP 3-219900 A (Otsuka Foods Co., Ltd.), 27 September 1991 (27.09.1991), page 3, upper left column, line 12 to upper right column, line 8 (Family: none) | 1-10 |
| A | JP 9-262098 A (Nichirei Corp.), 07 October 1997 (07.10.1997), paragraphs [0002] to [0006] (Family: none) | 1-10 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 62050120 B
- JP HEI9262098 A
- JP HEI8131196 A
- JP 2000051394 T
- WO 2002004633 A
- JP 2008206491 A
- JP 62050120 A
- JP 2000513940 T